# EUROPEAN PATENT SPECIFICATION

(11) **EP 4 118 229 B1**
(45) Date of publication and mention of the grant of the patent: **02.12.2026**
(21) Application number: 21767676.6
(22) Date of filing: 08.03.2021
(51) Int. Cl.: G16B 20/00, G16B 40/20, C12Q 1/68

(54) **MULTI-MODAL METHODS AND SYSTEMS**
MULTIMODALE VERFAHREN UND SYSTEME
PROCÉDÉS ET SYSTÈMES MULTIMODAUX

(30) Priority: 09.03.2020 US 202062986875 P; 08.09.2020 US 202063075691 P
(43) Date of publication of application: 18.01.2023
(73) Proprietor: Pioneer Hi-Bred International, Inc., Johnston, Iowa 50131-1014 (US)
(72) Inventor: JIA, Yi, Indianapolis, Indiana 46268 (US)
(74) Representative: J A Kemp LLP
(86) International application number: PCT/US2021/021282
(87) International publication number: WO 2021/183408

(56) References cited:
- US-A1- 2011 296 753
- US-A1- 2014 220 568
- US-A1- 2016 080 665
- US-A1- 2019 244 680
- US-A1- 2019 244 680
- HARFOUCHE ANTOINE L ET AL: "Accelerating Climate Resilient Plant Breeding by Applying Next-Generation Artificial Intelligence", TRENDS IN BIOTECHNOLOGY, ELSEVIER PUBLICATIONS, CAMBRIDGE, GB, vol. 37, no. 11, 21 June 2019 (2019-06-21), pages 1217 - 1235, XP085889830, ISSN: 0167-7799, [retrieved on 20190621], DOI: 10.1016/J.TIBTECH.2019.05.007
- WANG SHIBO ET AL: "Identification of optimal prediction models using multi-omic data for selecting hybrid rice", HEREDITY, SPRINGER INTERNATIONAL PUBLISHING, CHAM, vol. 123, no. 3, 25 March 2019 (2019-03-25), pages 395 - 406, XP036861486, ISSN: 0018-067X, [retrieved on 20190325], DOI: 10.1038/S41437-019-0210-6
- CUDIC MIHAEL ET AL: "Prediction of Sorghum Bicolor Genotype from In-Situ Images Using Autoencoder-Identified SNPs", 2018 17TH IEEE INTERNATIONAL CONFERENCE ON MACHINE LEARNING AND APPLICATIONS (ICMLA), IEEE, 17 December 2018 (2018-12-17), pages 23 - 31, XP033502253, DOI: 10.1109/ICMLA.2018.00012

## Description

### CROSS REFERENCE TO RELATED APPLICATIONS

### FIELD

This disclosure relates generally to the fields of prediction, phenotypic analysis, and breeding.

### BACKGROUND

Over the last 60 to 70 years, the contribution of plant breeding to agricultural productivity has been spectacular (Smith (1998) 53rd Annual corn and sorghum research conference, American Seed Trade Association, Washington, D.C.; Duvick (1992) Maydica 37: 69). This has happened in large part because plant breeders have been adept at assimilating and integrating information from extensive evaluations of segregating progeny derived from multiple crosses of elite, inbred lines. Conducting such breeding programs requires extensive resources. A commercial maize breeder, for example, may evaluate 1,000 to 10,000 F3 topcrossed progeny derived from 100 to 200 crosses in replicated field trials across wide geographic regions. Therefore, plant breeders are interested in developing high yielding varieties and agronomically sound hybrids using less resources. Further, plant breeders are challenged with continually increasing the performance of their products to help meet the growing demand and future needs for food and feed supplies.

HARFOUCHE ANTOINE L ET AL: "Accelerating Climate Resilient Plant Breeding by Applying Next-Generation Artificial Intelligence", TRENDS IN BIOTECHNOLOGY, ELSEVIER PUBLICATIONS, CAMBRIDGE, GB, vol. 37, no. 11, pages 1217-1235 discusses the state of genomics and field phenomics as well as emerging approaches and challenges for multiomics big data integration by means of artificial intelligence.

### SUMMARY

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

Provided herein are methods of predicting at least one phenotype of interest in one or more organisms. In some aspects, the methods include generating an universal integrated latent space representation by encoding variables derived from two or more types of data into latent vectors through an autoencoder. The two or more types of data includes but is not limited to genomic data, exomic data, epigenomic data, transcriptomic data, proteomic data, metabolomic data, hyperspectral data, or phenomic data, or combinations thereof. The latent space is independent of the underlying genomic, exomic, epigenomic, transcriptomic, proteomic, metabolomic, hyperspectral, or phenomic association. The method also includes decoding the integrated latent representation by a decoder to obtain reconstructed data. The input data may be obtained from a training population or data set, testing population or data set, or both. In some aspects, the method also includes inputting the reconstructed data and observed phenotype data for at least one phenotype of interest obtained from the training population to train a supervised learning model. At least one phenotype of interest for one or more organisms from the testing population may be predicted by inputting the reconstructed data for the testing population into the trained supervised learning model. In some aspects, the organism is a microorganism, an animal, or a plant. In some aspects, the generated universal integrated latent space representation is continuous. In some aspects, the integrated latent space representation may be generated by encoding discrete, continuous, or combined variables derived from two or more different types of data into latent vectors using an autoencoder. In some examples, the autoencoder is a multi-modal autoencoder. In some examples, the autoencoder is a variational autoencoder. In some examples, the autoencoder is a multi-modal variational autoencoder. In some examples, the input data is genomic, exomic, epigenomic, transcriptomic, proteomic, metabolomic, hyperspectral, or phenomic, which includes but is not limited to genomic-wide data, exomic-wide data, epigenomic-wide data, transcriptomic-wide data, proteomic-wide data, metabolomic-wide data, hyperspectral data, phenomic data, or combinations thereof.

Also provided herein is a computer system, device, or readable medium for generating phenotypic determinations. In one embodiment, the system includes a neural network that includes an autoencoder configured to encode genomic, exomic, epigenomic, transcriptomic, proteomic, metabolomic, hyperspectral, and/or phenomic data information from two or more types of input data obtained from training and testing populations, where the two or more types of input data comprises genomic, exomic, epigenomic, transcriptomic, proteomic, metabolomic, hyperspectral, and/or phenomic data into universal multi-modal latent vectors, where the autoencoder has been trained to represent two or more types of data comprising genomic, exomic, epigenomic, transcriptomic, proteomic, metabolomic, hyperspectral, and/or phenomic data associations and includes a decoder configured to decode the encoded latent vectors and generate reconstructed input data and a second neural network or other machine learning algorithm that includes a supervised learning model configured to predict at least one phenotype of interest for one or more organisms, such as plants, animals, or microorganisms. The supervised learning model may be trained for prediction determinations using as input the reconstructed input data from the training population and observed phenotype data for at least one phenotype of interest obtained from the training population or data set. In some aspects, the trained supervised learning model may be used to predict a phenotype for one or more organisms from the testing population by receiving as input the reconstructed data for the testing population. In some aspects, the autoencoder may be a multi-modal autoencoder, a variational autoencoder, or a multi-modal variational autoencoder.

Use of the methods and systems described herein may be used to predict phenotypes and aid in the culling of certain undesirable traits or phenotypes or organisms, such as plants, from a breeding program. Alternately or addition to, the methods and systems described herein may be used to predict phenotypes and aid in the selection of or advancement of certain desirable traits or phenotypes or organisms, such as plants, in a breeding program.

### BRIEF DESCRIPTION OF THE DRAWINGS

The invention can be more fully understood from the following detailed description and the accompanying drawings which form a part of this application.

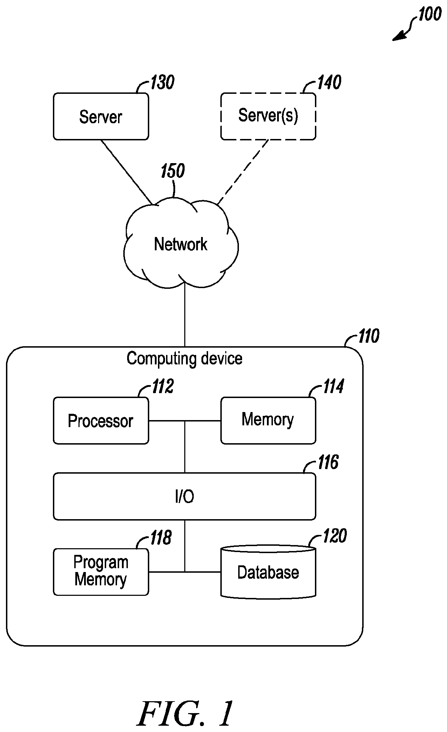

FIG. 1 is a block diagram illustrating an exemplary computer system including a server and a computing device according to an embodiment as disclosed herein;

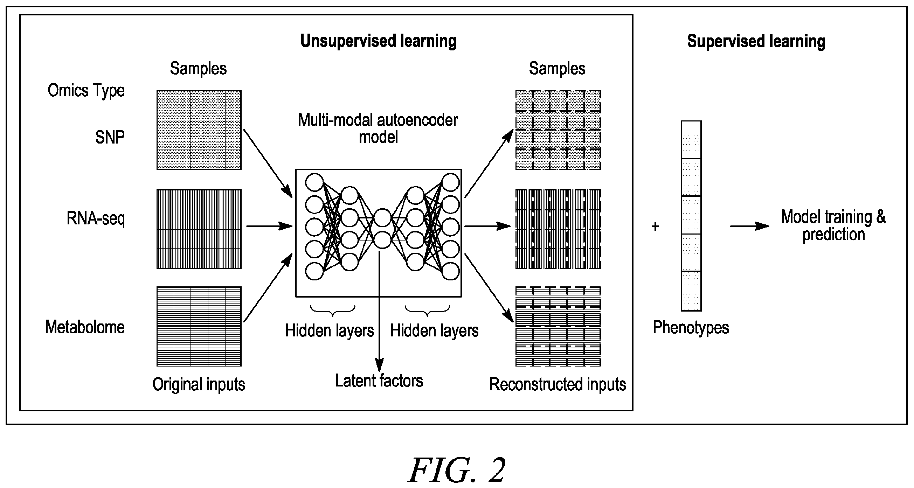

FIG. 2 is a schematic illustrating the use of unsupervised and supervised learning framework for phenotype prediction with multi-omics data integration in one embodiment. Three types of omics data as examples on a panel of samples are fed into multi-modal autoencoder model to infer the latent factors representing the three types of multi-omics data. Then this multi-omics autoencoder model is trained to reconstruct input data after passing the input data through the bottleneck layer (i.e. latent vector). Such reconstructed multi-omics data with observed phenotype data for at least one phenotype of interest (e.g. breeding traits) from the training population serve as the input data of supervised learning or prediction evaluation (e.g. genomic selection).

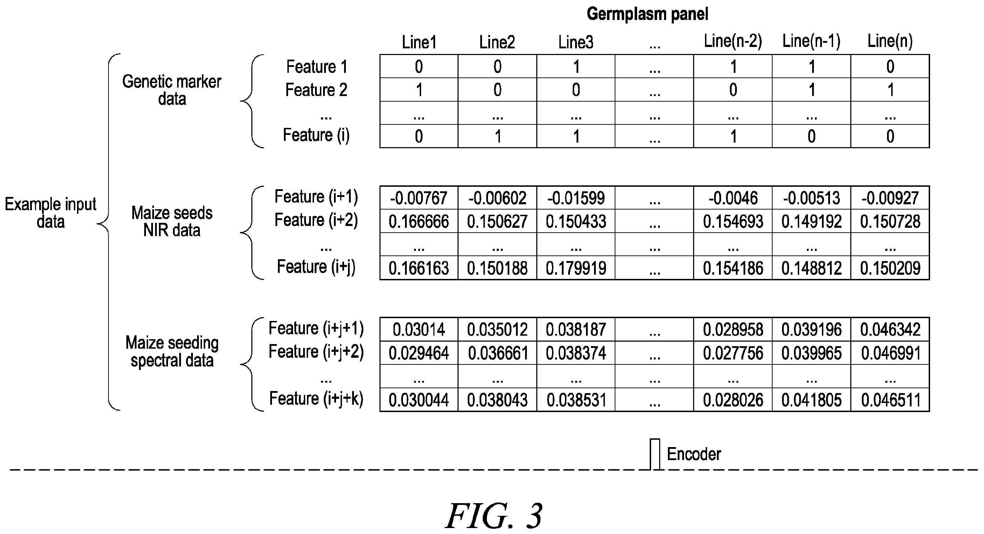

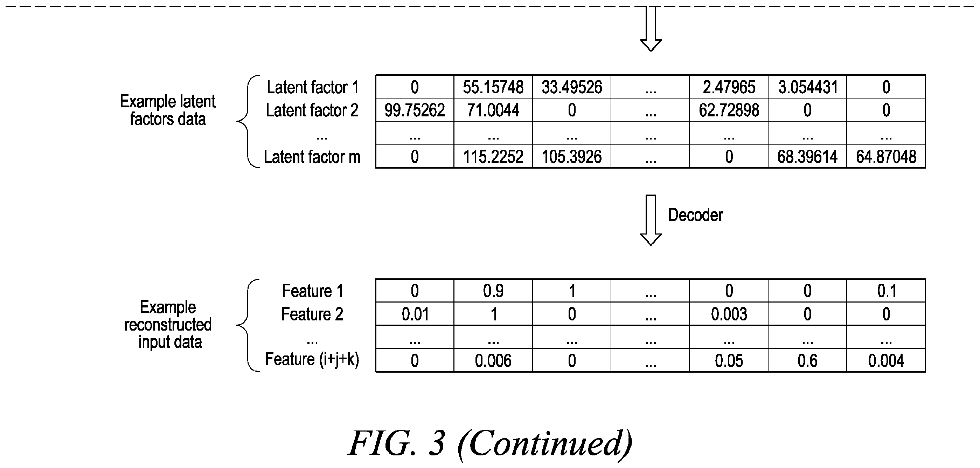

FIG. 3 is an example of input, latent factor and reconstructed input through the unsupervised learning framework in one embodiment.

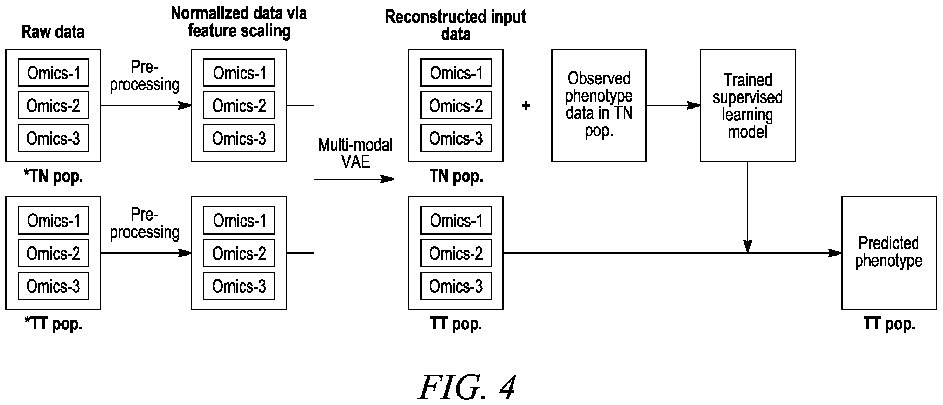

FIG. 4 is a diagram illustrating the various steps in one embodiment of a phenotype prediction method. Raw data of three data types from a training population and a testing population is pre-processed via feature scaling, and the normalized data integrated into a multi-modal VAE which outputs the reconstructed input data from the training and testing populations. The reconstructed input data from the training population and observed phenotype data for at least one phenotype of interest from the training population is used to train a supervised learning model. The reconstructed input data from the testing population is inputted into the trained supervised learning model to predict a phenotype of interest for a member or population of the testing population. TN pop.: training population; TT pop.: Testing population.

### DETAILED DESCRIPTION

It is to be understood that this invention is not limited to particular embodiments, which can, of course, vary. It is also to be understood that the terminology used herein is for the purpose of describing particular embodiments only, and is not intended to be limiting. Further, all publications referred to herein are each incorporated by reference for the purpose cited to the same extent as if each was specifically and individually indicated to be incorporated by reference herein.

Applying the phenotype prediction methods and systems disclosed herein to a breeding program allows a breeder to decide which plants should be advanced and which plants should be culled from a breeding program without having to physically grow the plants to determine a plant's phenotype, thereby providing savings in time, finances, laboratory and field resources, and labor.

In some aspects, use of the disclosed methods and systems herein may generate better prediction performance that may be used to increase parent selection accuracy, increase selection intensity, or both so fewer parents need to be selected in the breeding program while allowing the rate of genetic gain to accelerate. Accordingly, use of the prediction methods and systems in a plant breeding program may accelerate the rate of genetic gain for germplasm improvement through parental selection and advancement.

The disclosed methods and systems herein take advantage of both unsupervised and supervised learning by integrating the unsupervised and supervised learning framework together for phenotype prediction with better performance. The unsupervised learning takes the feature data from both training and testing populations without phenotype or labels as input data to reconstruct the input data from the latent space which infers the underlying relationship between training and testing populations. In some embodiments, the supervised learning takes two input data from a training population: 1) reconstructed feature input data derived from the unsupervised learning procedure; and 2) observed phenotypes. The supervised learning maps a function from feature data to phenotype. The mapped function is applied with the reconstructed feature data from the testing population derived from the unsupervised learning for phenotype prediction on individuals in the testing population. The better prediction performance for the testing population may be partially attributed to, in some aspects, the reconstructed input feature data for the testing population learned from both training and testing populations.

In some aspects, the disclosed methods and systems herein improve upon modeling and learning the underlying relationship between training and testing populations and improve upon mapping a function from features to phenotypes. The phenotypes observed on living organisms are controlled and regulated by complex cellular processes. Different cellular processes relate to different biomolecules at different levels. Thus, the multi-omics or multi-modal data capture biological signals in multiple levels of cellular processes and may capture in some examples two different benefits: 1) In the unsupervised learning, the multi-omics or multi-modal data from both training and testing populations are applied as the input data to output the reconstructed multi-omics or multi-modal input data through multi-modal variational autoencoder learning. The unsupervised learning with joint multi-omics or multi-modal data from both training and testing populations can model and learn the underlying relationship better between the two population than single-omics data only. 2) The multi-omics can capture multiple cellular processes to map a better function from features to phenotypes.

Further, methods and systems provided herein minimize the labor intensive steps normally associated with machine learning application such as for example, the construction of a feature set that is relevant for the scope of the problem, and satisfaction of the constraints of the algorithm(s) to be used.

Referring to FIG. 1, a block diagram of a computer system 100 for parametrically representing multi-omics or multi-modal association data is shown. To do so, the system 100 may include a computing device 110 and a server 130 that is associated with a computer system. The system 100 may further include one or more servers 140 that are associated with other computer systems such that the computing device 110 may communicate with different computer systems running different platforms. However, it should be appreciated that, in some embodiments, a single server (e.g., a server 130) may run multiple platforms. The computing device 110 is communicatively coupled to the one or more servers 130, 140 via a network 150 (e.g., a local area network (LAN), a wide area network (WAN), a personal area network (PAN), the Internet, etc.).

In use, the computing device 110 may predict multi-omics or multi-modal associations by a neural network receiving two or more types of multi-omics or multi-modal data. The multi-omics or multi-modal data may include combinations of genomic, exomic, epigenomic, transcriptomic, proteomic, metabolomic, and/or phenomic data, such as hyperspectral imaging, which includes but is not limited to genomic-wide data, exomic-wide data, epigenomic-wide data, transcriptomic-wide data, proteomic-wide data, metabolomic-wide data, and phenomic-data, such as hyperspectral data, or combinations thereof.

More specifically, the computing device 110 may obtain multi-omics or multi-modal data and translate the data into a universal latent space that is independent of the underlying multi-omics or multi-modal data. For example, in the context of trait prediction, a smooth spatial organization of the latent space captures underlying correlations that are present within a multi-omics or multi-modal dataset. As described further below, variational autoencoders (VAEs) may be used to compress the information contained within a multi-omics or multi-modal data set to a common, multi-omics-invariant or multi-modal-invariant, latent space capable of capturing these underlying correlations.

In general, the computing device 110 may include any existing or future devices capable of training a neural network. For example, the computing device may be, but not limited to, a computer, a notebook, a laptop, a mobile device, a smartphone, a tablet, wearable, smart glasses, or any other suitable computing device that is capable of communicating with the server 130.

The computing device 110 includes a processor 112, a memory 114, an input/output (I/O) controller 116 (e.g., a network transceiver), a memory unit 118, and a database 120, all of which may be interconnected via one or more address/data bus. It should be appreciated that although only one processor 112 is shown, the computing device 110 may include multiple processors. Although the I/O controller 116 is shown as a single block, it should be appreciated that the I/O controller 116 may include a number of different types of I/O components (e.g., a display, a user interface (e.g., a display screen, a touchscreen, a keyboard), a speaker, and a microphone).

The processor 112 as disclosed herein may be any electronic device that is capable of processing data, for example a central processing unit (CPU), a graphics processing unit (GPU), a system on a chip (SoC), or any other suitable type of processor. It should be appreciated that the various operations of example methods described herein (i.e., performed by the computing device 110) may be performed by one or more processors 112. The memory 114 may be a random-access memory (RAM), read-only memory (ROM), a flash memory, or any other suitable type of memory that enables storage of data such as instruction codes that the processor 112 needs to access in order to implement any method as disclosed herein. It should be appreciated that, in some embodiments, the computing device 110 may be a computing device or a plurality of computing devices with distributed processing.

As used herein, the term "database" may refer to a single database or other structured data storage, or to a collection of two or more different databases or structured data storage components. In the illustrative embodiment, the database 120 is part of the computing device 110. In some embodiments, the computing device 110 may access the database 120 via a network such as network 150. The database 120 may store data (e.g., input, output, intermediary data) that is necessary to generate a universal continuous integrated latent space representation. For example, the data may include two or more types of genomic, exomic, epigenomic, transcriptomic, proteomic, metabolomic, hyperspectral, or phenomic data that are obtained from one or more servers 130, 140. In some examples, the data includes genomic data, transcriptomic data, proteomic, metabolomic, hyperspectral, and/or phenomic data or any combination thereof.

The computing device 110 may further include a number of software applications stored in a memory unit 118, which may be called a program memory. The various software applications on the computing device 110 may include specific programs, routines, or scripts for performing processing functions associated with the methods described herein. Additionally or alternatively, the various software applications on the computing device 110 may include general-purpose software applications for data processing, database management, data analysis, network communication, web server operation, or other functions described herein or typically performed by a server. The various software applications may be executed on the same computer processor or on different computer processors. Additionally, or alternatively, the software applications may interact with various hardware modules that may be installed within or connected to the computing device 110. Such modules may implement part of or all of the various exemplary method functions discussed herein or other related embodiments.

Although only one computing device 110 is shown in FIG. 1, the server 130, 140 is capable of communicating with multiple computing devices similar to the computing device 110. Although not shown in FIG. 1, similar to the computing device 110, the server 130, 140 also includes a processor (e.g., a microprocessor, a microcontroller), a memory, and an input/output (I/O) controller (e.g., a network transceiver). The server 130, 140 may be a single server or a plurality of servers with distributed processing. The server 130, 140 may receive data from and/or transmit data to the computing device 110.

The network 150 is any suitable type of computer network that functionally couples at least one computing device 110 with the server 130, 140. The network 150 may include a proprietary network, a secure public internet, a virtual private network and/or one or more other types of networks, such as dedicated access lines, plain ordinary telephone lines, satellite links, cellular data networks, or combinations thereof. In embodiments where the network 150 comprises the Internet, data communications may take place over the network 150 via an Internet communication protocol.

In some aspects, the universal continuous integrated latent space representation may be generated by encoding discrete or continuous variables or combinations of both derived from two or more types of data into latent vectors using an autoencoder, for example, a VAE, a multi-modal autoencoder (MAE), or a multi-modal variational autoencoder (MVAE).

The core of VAE is rooted in Bayesian inference, which includes modeling of the underlying probability distribution of data, such that new data can be sampled from that distribution, which is independent of the dataset that resulted in the probability distribution. VAEs have a property that separates them from standard autoencoders that is suitable for generative modeling: the latent spaces that VAEs generate are, by nature of the framework, probability distributions, thereby allowing simpler random sampling and interpolation for desirable end-uses. VAEs accomplish this latent space representation by making its encoder not output an encoding vector of size n, rather, outputting two vectors of size n: a vector of means, µ, and another vector of standard deviations, σ. Some of the basic notions for VAE include for example:
X: data that needs to be modeled, for example, genotypic data (such as SNPs, markers, haplotype, sequence information); transcriptomic data (such as genes); metabolomic data (such as metabolites), exomic data, proteomic data (such as proteins), hyperspectral imaging data (such as wavelengths) and so on. The X data can be from one type or combinations of types listed above.
z: latent variable
P(X): probability distribution of the data, for example, genotypic data
P(z): probability distribution of latent variable (e.g., genotypic associations from the underlying genotypic data)
P(X|z): distribution of generating data given latent variable, e.g. prediction, imputation or reconstruction of the desired outcome based on the latent variable.

VAE is based on the principle that if there exists a hidden variable z, which generates an observation or an outcome x, then one of the objectives is to model the data, i.e., to find P(X). However, one can observe x, but the characteristics of z need to be inferred. Thus, p(z|x) needs to be computed. $p \left(\left.z\right|x\right) = p \left(\left.x\right|z\right) p \left(z\right) / p \left(x\right)$

However, computing p(x) is based on probability theory, in relation to z. This function can be expressed as follows: $p \left(x\right) = \int p \left(\left.x\right|z\right) p \left(z\right) dz$

While the p(x) function is an intractable distribution, variational inference is used to optimize the joint distribution of x and z. The function p(z|x) is approximated by another distribution q(z|x), which is defined such that it is a tractable distribution. The parameters of q(z|x) are defined such that they are highly similar to p(z|x) and therefore, it can be used to perform approximate inference of the intractable distribution. KL divergence is a measure of difference between two probability distributions. Therefore, if the goal is to minimize the KL divergence between the two distributions, this minimization function is expressed as: $min KL \left(\left.q\left(\left.z\right|x\right)\right‖p\left(\left.z\right|x\right)\right)$

This expression is minimized by maximizing the following: $Eq \left(\left.z\right|x\right) log p \left(\left.x\right|z\right) - KL \left(\left.q\left(\left.z\right|x\right)\right‖p\left(z\right)\right)$

Reconstruction likelihood is represented by the first part, and the second term penalizes departure of probability mass in q from the prior distribution, p. q is used to infer hidden variables (latent representation) and this is built into a neural network architecture where the encoder model learns the mapping relation from x to z and the decoder model learns the mapping from z back to x. Therefore, the neural network for this function includes two terms - one that penalizes reconstruction error or maximizes the reconstruction likelihood and the other that encourages the learned distribution q(z|x) to be highly similar to the true prior distribution p(z), which is assumed to follow a unit Gaussian distribution, for each dimension j of the latent space. This is represented by: $L \left(x, \hat{x}\right) + {\sum }_{j} KL \left(\left.{q}_{j}\left(\left.z\right|x\right)\right‖p\left(z\right)\right)$

It should be appreciated that the variational autoencoder is one of several techniques that may be used for producing compressed latent representations of raw samples, for example, genotypic association data. Like other autoencoders, the variational autoencoder places a reduced dimensionality bottleneck layer between an encoder and a decoder neural network. Optimizing the neural network weights relative to the reconstruction error then produces separation of the samples within the latent space. However, unlike generative adversarial networks (GAN), the encoder neural network's outputs are parameterized univariate Gaussian distributions with standard N(0,1) priors. Thus, unlike other autoencoders, which tend to memorize inputs and place them in arbitrarily small locations within the latent space, the variational autoencoder produces a smooth, continuous latent space in which semantically-similar samples tend to be geometrically close.

Some VAEs are uni-modal in distribution, while other VAEs model more than one mode of data distributions, i.e, multi-modal distributions, e.g. bi- or tri-modal, and are referred to as multi-modal VAEs (MVAEs). MVAEs are able to learn and model multiple modes of data distributions. Multi-modal latent variables in MVAEs may be learned by any suitable approach, for example, by using a mixture of Gaussians (e.g. multivariate Gaussians) or models with both discrete and continuous latent variables.

Any suitable multi-modal autoencoder (MMAE) or MVAE that is able to take multiple layers of information as input with normalization through pre-processing may utilized in the methods and compositions described herein. The MMAE or MVAE may use the below or similar formula/equation to output a reconstruct the input data from multiple layers of data sources.$L = - {E}_{q \left(\left.z\right|x\right)} \left[log\left(p\left(\left.x\right|z\right)\right)\right] + {D}_{KL} \left(\left.q\left(\left.z\right|x\right)\right‖p\left(z\right)\right)$

When a multi-modal variational autoencoder is used, the latent vector is subjected to a probabilistic distribution constraint.

In some aspects, the method includes generating a universal continuous integrated latent space representation by encoding discrete or continuous variables or a combination of both types of variables derived from two or more data sets from same or different types of data into latent vectors through a machine learning-based encoder framework. Non-limiting examples of data comprise genomic, exomic, epigenomic, transcriptomic, proteomic, metabolomic, hyperspectral, and/or phenomic data. In some aspects, the method includes encoding by the encoder, genomic, exomic, epigenomic, transcriptomic, proteomic, metabolomic, hyperspectral, or phenomic information from two or more types of data comprising genomic, exomic, epigenomic, transcriptomic, proteomic, metabolomic, hyperspectral, or phenomic data into latent vectors. In some aspects, the method includes training the multi-modal encoder using the latent vectors to learn underlying genomic, exomic, epigenomic, transcriptomic, proteomic, metabolomic, hyperspectral, and/or phenomic correlations and/or relatedness.

In some examples, the encoder is an autoencoder. In some examples, the encoder is a MMAE. In some examples, the autoencoder is a VAE. In some examples, the autoencoder is a MVAE. In some aspects, the machine-learning based encoder framework is a generative adversarial network (GAN) such as a multi-modal GAN (MMGAN). In some aspects, the machine-learning based encoder framework is a neural network. In some examples, the MVAE is a machine learning-based multi-modal variational autoencoder. In some aspects, the machine-learning based encoder framework is a neural network.

In some aspects, the latent space is independent of the underlying genomic, exomic, epigenomic, transcriptomic, proteomic, metabolomic, hyperspectral imaging, or phenomic association used to represent the genomic, exomic, epigenomic, transcriptomic, proteomic, metabolomic, hyperspectral, or phenomic information. For example, the generated latent representations are invariant to the selection of particular genomic, exomic, epigenomic, transcriptomic, proteomic, metabolomic, hyperspectral, or phenomic association features. In some aspects, the latent space is independent of the underlying associated features in input data. As used herein, "feature" or "associated data feature" generally refers to a property or characteristic of an observation in a dataset. Examples of features or associated data features for different types of -omic data, include, for example, SNPs for genotype data; genes for transcriptome data; and wavelength for hyperspectral data.

In some aspects, the method includes providing the encoded multi-modal latent vectors or representation to decoder. In some aspects, the decoder is neural network.. In some aspects, the decoder is an autodecoder. In some aspects, the decoder is a multi-modal autodecoder. In some aspects, the decoder is a variational autodecoder. In some aspects, the decoder is a multi-modal variational autodecoder. In some aspects, the method includes training the decoder to reconstruct the multi-modal input data based on a pre-specified or learned objective function. In some aspects, the method includes decoding by the decoder the encoded multi-modal latent vector for the objective function. In some aspects, the method includes providing an output for the objective function of the decoded latent vector. In some aspects, the method includes decoding the latent representation by a decoder, thereby reconstructing the multi-modal input data. In some aspects, the method includes using the reconstructed input data from the testing population in predicting the phenotype of interest for one or more organisms from the testing population.

A VAE or other autoencoder, such as a MMAE or MVAE, may be trained with inputs from two or more types of multi-omics or multi-modal data. The inputs may include combinations of genomic, exomic, epigenomic, transcriptomic, proteomic, metabolomic, hyperspectral, or phenomic data. In some examples, the input data is genomic, exomic, epigenomic, transcriptomic, proteomic, metabolomic, hyperspectral, and/or phenomic, which includes but is not limited to genomic-wide data, exomic-wide data, epigenomic-wide data, transcriptomic-wide data, proteomic-wide data, metabolomic-wide data, hyperspectral data, and/or phenomic-data or combinations thereof.

The inputs may include data from one or more training populations or data sets, testing populations or data sets, or both. In some examples, the training population or data set and testing population or data set are from plants, such as inbred or hybrid plants. In some aspects, the plant data is obtained from plants, parts thereof, or both, including but not limited to seeds or seedlings. In some aspects, the plant data is obtained from plants or parts thereof from a field, a greenhouse, laboratory, or combinations thereof.

Also provided herein, in one embodiment, is a universal method of parametrically representing genomic, exomic, epigenomic, transcriptomic, proteomic, metabolomic, hyperspectral, or phenomic association data from a data set obtained from one or more training and testing populations or data sets to reconstruct the feature data.

In some aspects, the method includes decoding the latent representation by a multi-modal decoder, thereby reconstructing the feature data in training and testing data by the decoded latent representation. In some aspects, the method includes decoding the latent representation by a multi-modal decoder, thereby reconstructing the multi-modal input data. Any suitable decoder may be used in the methods and compositions described herein, for example, those in VAEs, MMAEs, or MVAEs, and trained to decode the encoded latent vectors from the encoder and reconstruct the inputs from the multi-omics or multi-modal data.

The universal latent representations may be used in methods such as phenotype prediction. In some aspects, the methods include generating an integrated latent representation by encoding a subset of the discrete or continuous or a combination of both types of variables derived from the two or more types of data set into latent vectors through a machine learning-based autoencoder and compressing the information contained within the given data set, e.g. obtained from training and testing populations, to a common latent space to create a latent representation of that information.

In at least one embodiment, a MMAE such as a MVAE may receive as input information from two or more types of data and encode the information into a latent representation. The latent representation is decoded by a decoder into uncompressed form as output, for example, reconstructed multi-omics input data. See, for example, FIG. 3 and Example 3, where a decoder decoded the latent representations as reconstructed multi-omics data. Some or all of the input data may be preprocessed prior to being encoded, such as scaled, for example, feature scaling, standardized, normalized, generalized, and/or regularized.

In one embodiment, in the training stage, the multi-modal autoencoder, which includes an encoder trained to encode the input data (the features from the input data) obtained from the training and testing populations into a latent representation in the latent space and a decoder to decode the latent representation and to reconstruct the input data using unsupervised learning. In some aspects, the decoder is trained on existing genomic, exomic, epigenomic, transcriptomic, proteomic, metabolomic, hyperspectral, or phenomic data, or combinations thereof. The training of the autoencoder to learn the encoding and decoding of the two or more types of data or multi-omics data may be iterative until the likelihood of reconstructing the input data reaches a certain level or threshold of accuracy.

In at least one embodiment, a neural network or an autoencoder with an encoder and decoder is trained using unsupervised learning or any other suitable technique. Unsupervised learning is a method that may be used for training the autoencoder in a state in which a label is not allocated to training data. Unsupervised learning includes the task of trying to find hidden structure in unlabeled data. Some examples of unsupervised learning processes include but are not limited to: clustering (e.g., k-means, mixture models, hierarchical clustering), blind signal separation using feature extraction techniques for dimensionality reduction (e.g., principal component analysis, independent component analysis, non-negative matrix factorization, singular value decomposition) and artificial neural networks (e.g., self-organizing map, adaptive resonance theory). Clustering analysis is the assignment of a set of observations into subsets (called clusters) so that observations within the same cluster are similar according to some predesignated criterion or criteria, while observations drawn from different clusters are dissimilar. Different clustering techniques make different assumptions on the structure of the data, often defined by some similarity metric and evaluated for example by internal compactness (similarity between members of the same cluster) and separation between different clusters. An unsupervised algorithm, e.g., a clustering or dimensionality reduction algorithm, may find previously unknown patterns in data sets without pre-existing labels. Accordingly, in at least one embodiment, the untrained autoencoder trains itself using unlabeled data. In some aspects, the unsupervised learning training dataset includes two or more types of input data, such as multi-omics or multi-modal data, without any associated output data. The training data set may be obtained from a training population, a testing population, or both. The autoencoder through training using unsupervised learning becomes capable of finding hidden structure in unlabeled data, learning groupings within training dataset, determining how individual inputs are related to the untrained dataset, or combinations thereof.

The details of the network structure and the training approach are readily adapted or adjusted to suit any particular application. For instance, convolutional neural networks for encoders and/or decoders may be used in to enforce known spatial structure on hidden layer representations. In some aspects, a Long Short-Term Memory (LSTM) autoencoder may be used in to enforce sequence data using an encoder-decoder LSTM architecture.

In at least one embodiment, the decoder in the multi-modal autoencoder is trained through unsupervised learning to reconstruct feature input data after passing the feature input data through the bottleneck layer (i.e. latent vector). The bottleneck layer may include a fewer number of nodes than the one or more preceding hidden layers of the neural network. The bottleneck layer may create a constriction to learn the feature data through a representation of compressed data. The unsupervised learning may include training the model with the feature input. In some embodiments, the method of predicting a phenotype includes a model trained to predict phenotypes through supervised learning using the reconstructed feature input and an observable phenotype as input. In some aspects, the reconstructed feature input is obtained from the training population. In some aspects, the observable phenotype data is obtained from the training population.

FIG. 2 is a schematic diagram illustrating one embodiment of the use of unsupervised and supervised machine learning framework for phenotype prediction with multi-omics data integration. Although three omics data types are used as examples in FIG. 2, it is understood that the number of omics types may be two, three, four, five, six, seven or more omics data types. In some embodiments, the two or more types of omics data, e.g. data from two or more types of genomic, exomic, epigenomic, transcriptomic, proteomic, metabolomic, hyperspectral, or phenomic data, are input into a multi-modal autoencoder model to infer the latent factors representing all layers of the multi-omics data. The terms autoencoder model, autoencoder algorithm, and autoencoder are used herein interchangeably.

In some aspects, the method includes receiving by a supervised learning model reconstructed input data of two or more types of genomic, exomic, epigenomic, transcriptomic, proteomic, metabolomic, hyperspectral, or phenomic data from the decoder of the autoencoder, e.g. a multi-modal autoencoder. In some aspects, the supervised learning model is trained to predict a phenotype for an organism, such as a plant or microorganism, using as input the reconstructed input data from the training population and data for observed phenotype of interest obtained from the training population (e.g. breeding or agronomic traits).

Supervised learning is a method that may be used to train a model in which a label is allocated to training data. Supervised learning includes inferring a function from labeled training data or teaching a machine learning model to map an input to an output using examples found in labeled training data. The label may refer to a correct answer or result value that the model should infer when the training data is input to the model. Supervised learning may be performed through the analysis of training examples and/or data. For example, an untrained model may be trained using labeled training inputs, i.e., training inputs with known outputs, such as reconstructed multi-omics or multi-modal data with observed phenotype data for at least one phenotype of interest. The training inputs may be provided to an untrained model to generate a predicted output, such as a predicted phenotype for a trait. The training data includes an feature input data (e.g., a feature vector from the reconstructed multi-omics or multi-modal data from the training population and observed phenotype data for at least one phenotype of interest from the training population). Part of the supervised learning process includes analyzing the training data and producing an inferred function, which is called a classifier. The inferred function should predict the output value for any valid input. This requires the supervised learning process to generalize from the training data to new situations. Some examples of supervised learning processes include but are not limited to: artificial neural networks, boosting (meta-algorithm), Bayesian statistics, decision tree learning, decision graphs, inductive logic programming, Naive Bayes classifier, nearest neighbor algorithm and support vector machines.

Any suitable supervised learning algorithm, such as a regression-based mechanism, ordinary least squares, lasso, multi-task lasso, elastic net, multi-task elastic net, least angle regression, LARS lasso, orthogonal matching pursuit (OMP), Bayesian regression, naive Bayesian, logistic regression, stochastic gradient descent (SGD), neural networks, Perceptron, passive aggressive algorithms, robustness regression, Huber regression, polynomial regression, linear and quadratic discriminant analysis, kernel ridge regression, support vector machines, stochastic gradient descent, nearest neighbor, Gaussian processes, cross decomposition, decision trees, and/or ensemble methods may be used to learn a function to map an input to an output based on input-output pairs in training data sets. For example, the supervised learning model used to predict at least one phenotype of interest for one or more plants may be trained using ridge regression, LASSO, or other algorithms as described elsewhere herein and known to one skilled in the art. Ridge regression addresses some of the problems of Ordinary Least Squares by imposing a penalty on the size of the coefficients. The ridge coefficients minimize a penalized residual sum of squares: ${min}_{w} {\left‖Xw-y\right‖}_{2}^{2} + α {\left‖w\right‖}_{2}^{2}$

The complexity parameter α≥0 controls the amount of shrinkage: the larger the value of α, the greater the amount of shrinkage and thus the coefficients become more robust to collinearity. See, for example, Arthur EH and Robert WK. Ridge regression: biased estimation for nonorthogonal problems Technometrics. (2000).

In some examples, the supervised learning model is trained in a supervised manner using the reconstructed inputs from a training multi-omics or multi-modal dataset and observed phenotype data for at least one phenotype of interest from the training population and comparing resulting outputs, such as phenotype prediction against a set of expected or desired outputs. The accuracy of the model may be evaluated and errors propagated back through model to improve the model's training so that it generates correct answers more frequently and hits the desired level of accuracy. The error of a model is the difference between actual performance and modeled performance. In some cases, weights may be adjusted using a loss function and adjustment algorithm, such as stochastic gradient descent.

Accordingly, a value for the predicted phenotype of interest for one or more members of the testing population may be predicted. In at least one embodiment, the data, such as reconstructed input data from the training population and observed phenotype data for at least one phenotype of interest from the training population, is inputted into a model that is trained using supervised learning for use in predicting a phenotype of interest for an organism, such as a plant or microorganism. In some aspects, reconstructed input data for the testing population serves as the input data for predicting at least one or more phenotypes of interest for an organism in a trained supervised learning model.

In some embodiments, the data, such as feature input data, observed phenotype data for at least one phenotype of interest, is obtained from a training data set or population, a testing data set or population, or combinations thereof.

Examples of omics data includes but is not limited to genomic, exomic, epigenomic, transcriptomic, proteomic, metabolomic, or phenomic data, such as hyperspectral data. In some aspects, the genotypic data includes without limitation SNPs or indels or other sequence information. In some aspects, the genotypic data includes markers across the genome. In some aspects, the exomic data includes but is not limited to exome DNA sequences.

In some aspects, the epigenomic data includes but is not limited to gene expression, chromatin accessibility, DNA methylation, histone modifications, recombination hotspot, genomic landing locations for transgenes, transcription factor binding status data or any combination thereof. In some aspects, the transcriptomic data includes but is not limited to RNA transcript sequences and profile information. In some aspects, the proteomic data includes but is not limited to protein sequences and profile information. In some aspects, the metabolomic data includes but is not limited to metabolites and profile information. In some aspects, the phenomic data includes but is not limited to Red Green Blue (RGB) imaging data, infrared imaging data, or spectral imaging data, or any combinations thereof. Exemplary infrared imaging data includes but is not limited to near-infrared (NIR), far infrared (FIR), or thermal infrared imaging data and information or any combinations thereof. Exemplary spectral imaging includes but is not limited to hyperspectral or multispectral imaging data or information or any combinations thereof. In some aspects, the hyperspectral data includes but is not limited to wavelengths or bands and profile information. In some aspects, the multi-omics or multi-modal data includes sequence information from *in silico* crosses.

When the organism is a plant, the data may be obtained from any monocot or dicot plant, including but not limited to soybean, maize, sorghum, cotton, canola, sunflower, rice, wheat, sugarcane, alfalfa tobacco, barley, cassava, peanuts, millet, oil palm, potatoes, rye, or sugar beet plants. Accordingly, any monocot or dicot plant may used with the methods, compositions, and systems provided herein, including but not limited to a soybean, maize, sorghum, cotton, canola, sunflower, rice, wheat, sugarcane, alfalfa tobacco, barley, cassava, peanuts, millet, oil palm, potatoes, rye, or sugar beet plant. In some examples, the plants are inbred or hybrid plants.

In one embodiment, a method of predicting at least one phenotype of interest in one or more organisms, such as a plant, animal, or microorganism is provided. In some aspects, the phenotype is predicted for one or more members of populations of organisms, such as plant, animal, or microbial population. In some examples, the predicted phenotype is for an inbred or hybrid plant.

In one embodiment, a method of predicting at least one phenotype of interest for one or more organisms, such as a plant, animal, or microorganism, is provided. In some aspects, the method includes generating a universal integrated latent space representation by encoding discrete or continuous variables or combination of both types of variables derived from two or more types of data into latent vectors. In some aspects, the integrated latent space representation is a universal continuous integrated latent space representation. The data types may be the same or different. As an example, the data types may be the same type, e.g. all genomic, or different types, such as genomic and metabolomic. The data may include without limitation genomic, exomic, epigenomic, transcriptomic, proteomic, metabolomic, hyperspectral, or phenomic data, which includes but is not limited to genomic-wide data, exomic-wide data, epigenomic-wide data, transcriptomic-wide data, proteomic-wide data, metabolomic-wide data, hyperspectral data, and/or phenomic-data, or combinations thereof.

In at least one embodiment, the reconstructed data from the training population and observed phenotype data for at least one phenotype of interest from the training population are used as input in training the supervised learning model for the prediction of at least one phenotype of interest for an organism from a testing population, such as a plant or microorganism. As described herein, the systems and methods may be used to predict at least one phenotype of interest. The phenotype can be any phenotype of interest that may be predicted.

In some aspects, the observed phenotype and at least one predicted phenotype of interest is an agronomic phenotype, or a breeding trait, or combinations thereof. For example, in Example 3, the predicted phenotype for sixteen breeding traits is provided. In some aspects, the observed or predicted phenotype is yield. Non-limiting examples of the observed phenotype and predicted phenotype include yield, ear diameter, silking time, pollen shedding time, root lodging, stalk lodging, brittle snap, ear height, grain moisture, plant height, disease or pest resistance, abiotic stress tolerance, such as drought or salinity tolerance, test weight, predicted relative maturity, appearance of hybrids, covariate yield, stay green, canopy cover, hue of green color, canopy health, plot score green plant cover, plot score green hue or combinations thereof. In some aspects, the observed or predicted phenotype is a molecular phenotype including but not limited to gene expression, chromatin accessibility, DNA methylation, histone modifications, recombination hotspots, genomic landing locations for transgenes, transcription factor binding status, or a combination thereof.

In some examples, the methods include selecting one or more organisms, such as plants, based on the predicted phenotype of interest. The methods may include selecting one or more members of the testing population having a desired predicted value for the phenotype of interest. In some examples, the one or more selected plants are predicted to exhibit an improved or increased desirable phenotype of interest, such increased yield, increased drought resistance, or improved standability, as compared to a control plant or control plant population. A control plant or control plant population generally refers to a plant or plant population that is used as a comparative reference point for testing population plants for whom a phenotype of interest is predicted using the methods and system described herein. A control plant population, for example, may be a plant population of the same genotype as the testing population plants but when a phenotype of interest is predicted for the control plant population, the prediction method does not undergo the unsupervised multi-modal learning portion of the methods described herein and is only subjected to the trained supervised model portion with unimodal data as input, such as SNPs as input in ridge regression. See, for example, Examples 1 and 3 herein.

In some examples, the one or more selected plants, when grown, exhibit an improved or increased desirable phenotype of interest, such increased yield, increased drought resistance, or improved standability, as compared to a control plant or a control plant population. Accordingly, the methods may also include growing the selected plants or a part thereof in a plant growing environment, such as a greenhouse, a laboratory, a field, or any other suitable environment. The one or more organisms, such as one or more plants or microorganisms, including populations or one or more members thereof, that are predicted to have at least one desired phenotype of interest may be crossed with another plant or animal. When the organism is a plant, the selected member of the testing population may be bred with at least one other plant or selfed, e.g., to create a new line or hybrid, used in recurrent selection, bulk selection, or mass selection, backcrossed, used in pedigree breeding or open pollination breeding, and/or used in genetic marker enhanced selection. In some instances, a plant having at least one predicted desirable phenotype of interest may be crossed with another plant or back-crossed so that a desirable genotype may be introgressed into the plant by sexual outcrossing or other conventional breeding methods. In some examples, selected plants having at least one predicted desirable phenotype of interest may be used in crosses with another plant from the same or different population to generate a population of progeny. The plants may be grown and crossed according to any breeding protocol relevant to the particular breeding program. The one or more selected plants, progeny from crosses, or parts thereof may be used in a breeding program.

In some examples, the methods include selecting one or more organisms, such as plants, based on the predicted phenotype of interest. The methods may include selecting one or more members of the testing population having a undesired predicted value for the phenotype of interest. In some examples, the one or more selected plants exhibit an unimproved or less improved, poorer, or undesirable phenotype of interest, such decreased yield, increased drought susceptibility, or decreased standability, compared to a control plant or control plant population. Plants predicted to have at least one undesirable or less improved phenotype of interest, e.g. poorer yield, may be counter-selected and removed from a breeding program.

As desired, statistical methods such as best linear unbiased predictions (BLUP) may be used to cross-validate and compare the prediction accuracy of using multi-omics or multi-modal data types-based phenotype prediction methods and systems herein versus a SNP-based prediction method that does not utilize unsupervised learning. For example, in the SNP-based prediction method, genome-wide SNP data from maize lines without observed phenotype data may be used as testing input data for phenotype prediction using ridge regression. In one embodiment of multi-omics-based phenotype prediction, the multi-omics data as feature data from both training population and testing populations are trained in the multi-modal variational autoencoder unsupervised learning framework to output the reconstructed multi-omics input data for both training and testing populations. The observed phenotype data for at least one phenotype of interest obtained from the training population and reconstructed multi-omics inputs data from the training population are used to train in a supervised learning framework such as BLUP or ridge regression to predict at least one phenotype of interest for one or more plants or plant populations based on the reconstructed input data from the testing population. Accuracy may be calculated using the correlation between the predicted value and observed value for a specific phenotype on individuals in the testing population. See, for example, Examples 1 and 3, herein.

Also provided herein is a computer system for generating phenotypic predictions. In one embodiment, the system includes a first neutral network that includes an autoencoder configured to encode genomic, exomic, epigenomic, transcriptomic, proteomic, metabolomic, hyperspectral, or phenomic data information from two or more types of data obtained from training and testing populations comprising genomic, exomic, epigenomic, transcriptomic, proteomic, metabolomic, hyperspectral, and/or phenomic data into universal multi-modal latent vectors, where the encoder has been trained to represent two or more types of data comprising genomic, exomic, epigenomic, transcriptomic, proteomic, metabolomic, hyperspectral, or phenomic data associations and a decoder configured to decode the encoded latent vectors into reconstructed input data and generate an output for an objective function and a second neural network that includes a supervised learning model configured to predict at least one phenotype of interest for one or more organisms, such as plants or microorganisms, from the testing population using the reconstructed input data from the training population and observed phenotype data for at least one phenotype of interest obtained from the training population to train the supervised learning model. In some aspects, the encoder may be a MMAE. In some aspects, the autoencoder is a MVAE. The computer system may be configured or programmed to implement the methods described herein.

Provided herein is a method of predicting at least one phenotype of interest for one or more organisms, such as plants, comprising receiving by a first neural network two or more same or different types of input data obtained from a training population and a testing population, where the data comprises genomic, exomic, epigenomic, transcriptomic, proteomic, metabolomic, hyperspectral, and/or phenomic data, or combinations thereof, wherein the first neural network comprises a multi-modal autoencoder, wherein the autoencoder comprises a multi-modal autoencoder and a multi-modal autodecoder; encoding by the multi-modal autoencoder, the information from the two or more same or different types of data into latent vectors through a machine-learning based neural network training framework; training the decoder to learn to reconstruct the two or more same or different types of data (input data) using unsupervised learning; and receiving by a second neural network, wherein the second neural network comprises a supervised learning model, the reconstructed input data for the training population and observed phenotype data for at least one phenotype of interest obtained from the training population to train the model; and predicting the at least one phenotype of interest for one or more organisms, such as plants, from the testing population by inputting the reconstructed data from the testing population into the trained supervised learning model. The supervised learning model may be trained to learn to predict at least one phenotype of interest for one or more organisms, such as plants, from a testing population based on an objective function. In some aspects, the supervised learning model is trained to learn to predict at least one phenotype of interest using the reconstructed input data for the training population and observed phenotype data for at least one phenotype of interest obtained from the training population,

A computer system may be configured or programmed to implement the methods described herein. Also provided herein is a computing device comprising a processor configured to perform the steps of the methods herein. A computer-readable medium comprising instructions which, when executed by a computing device, cause the computing device to carry out the steps of any of the methods is provided herein.

Also provided herein in an embodiment is a universal method of parametrically representing two or more types of data comprising genomic, exomic, epigenomic, transcriptomic, proteomic, metabolomic, hyperspectral, or phenomic data obtained from a population or a data set from a plant or microorganism. In some aspects, the method includes generating a universal integrated latent space representation by encoding discrete or continuous variables or combination of both types of variables derived from two or more types of genomic, exomic, epigenomic, transcriptomic, proteomic, metabolomic, hyperspectral, and/or phenomic data into latent vectors through a machine learning-based multi-modal autoencoder framework, where the latent space is independent of the underlying genomic, exomic, epigenomic, transcriptomic, proteomic, metabolomic, hyperspectral, and/or phenomic data. In some aspects, the method includes decoding the latent representation by a multi-modal autodecoder into reconstructed input data.

Also provided herein is a computer system for generating latent representations from multiple types of data. In one embodiment, the system includes a first network that includes an encoder configured to encode genomic, exomic, epigenomic, transcriptomic, proteomic, metabolomic, hyperspectral, or phenomic data information from two or more types of data comprising genomic, exomic, epigenomic, transcriptomic, proteomic, metabolomic, hyperspectral, and/or phenomic data into universal multi-modal latent vectors, where the autoencoder has been trained to represent genomic, exomic, epigenomic, transcriptomic, proteomic, metabolomic, hyperspectral, and/or phenomic data associations through a machine-learning based network framework and a second network includes a decoder configured to decode the encoded latent vectors and generate an output for an objective function, such as reconstructed input data. In some aspects, the encoder may be an autoencoder. In some aspects, the autoencoder is a VAE, a MMAE, or a MVAE. In some aspects, the machine-learning based neural network framework is a generative adversarial network (GAN) such as a multi-modal GAN. In some aspects, the machine-learning based neural framework is a neural network.

Also provided herein is a system for training a neural network for predicting phenotypes. The system includes one or more servers and a computing device communicatively coupled to the one or more servers. Each of the one or more server storing different types of training and testing data associated with one or more populations. The computing device further includes a memory and one or more processors. The one or more processors are configured to obtain multi-omic or multi-modal training and testing data; generate an integrated latent space representation by encoding variables derived from the training and testing data into a set of latent vectors using a multi-modal encoder machine learning network; train a decoder machine learning network to decode one or more latent space representations to reconstruct the multi-omic or multi-modal training and testing data; and train a model using supervised learning to learn to predict at least one phenotype of interest for one or more plants from the reconstructed data from the training population data and observed phenotype data for at least one phenotype of interest from training data as input; and to predict at least one phenotype of interest for one or more plants from the testing population using the reconstructed data for the testing population as input.

Various types of general purpose or specialized computer systems and computing devices may be used with or perform operations in accordance with the teachings described herein. In some embodiments, the computer system 100 may solve problems that are highly technical in nature that cannot be performed as a set of mental acts by a human. Further, in certain embodiments, some of the processes performed may be performed by one or more specialized computers or computing devices to carry out defined tasks related to machine learning. In some embodiments, the computer system 100 and computing device 110 may be a specialized computer system or computing device configured for operating in a networked plant breeding program management system, platform, and/or architecture.

In one embodiment, provided herein are systems and methods of using an unsupervised and supervised learning framework with multi-omics or multi-modal data integration for the prediction of phenotypes. In some aspects, the methods are used in the selection of certain desirable traits or culling of certain undesirable traits or phenotypes of organisms, such as plants, in a breeding program. In some instances, use of the methods described herein increases the accuracy of the phenotypes predicted compared to a single omics approach, such as SNPs alone in a trained supervised model, e.g. ridge regression.

As used in this specification and the appended claims, terms in the singular and the singular forms "a," "an," and "the," for example, include plural referents unless the content clearly dictates otherwise. Thus, for example, reference to "plant," "the plant," or "a plant" also includes a plurality of plants; also, depending on the context, use of the term "plant" can also include genetically similar or identical progeny of that plant; use of the term "a nucleic acid" optionally includes, as a practical matter, many copies of that nucleic acid molecule; similarly, the term "probe" optionally (and typically) encompasses many similar or identical probe molecules.

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process, method, article, or apparatus that comprises a list of elements is not necessarily limited to only those elements but may include other elements not expressly listed or inherent to such composition, mixture, process, method, article, or apparatus.

As used herein, the term "haplotype" generally refers to the genotype of any portion of the genome of an individual or the genotype of any portion of the genomes of a group of individuals sharing essentially the same genotype in that portion of their genomes.

As used herein, the term "autoencoder" generally refers to a network that includes an encoder which takes in input and generates a representation (the encoding) of that information and a decoder which takes in the output of the encoder and reconstructs a desired output format. Autoencoders make the encoder generate encodings or representations that are useful for reconstructing its own/prior input and, the entire network may be trained as a whole with the goal of minimizing reconstruction loss.

As used herein, the term "encoder" generally refers to a network which takes in an input and generates a representation (the encoding) that contains information relevant for the next phase of the network to process it into a desired output format.

As used herein, the term "decoder" generally refers to a network which takes in the output of the encoder and reconstructs a desired output format.

Embodiments of the disclosure presented herein provide methods and compositions for using latent representations of data to predict information.

### EXAMPLES

The present invention is illustrated by the following examples. The foregoing and following description of the present invention and the various examples are not intended to be limiting of the invention but rather are illustrative thereof. Hence, it will be understood that the invention is not limited to the specific details of these examples.

### EXAMPLE 1: Integrative Multi-omics Application for Phenotype Prediction in Plant Breeding Using Genetic Markers, Transcriptome, and Metabolome

The hybrid framework of unsupervised and supervised learning illustrated in FIG. 4 was applied to a total of 339 maize lines with SNP, RNA-seq and metabolome data available from a public association panel. Among the multi-omics data, there are 72,572 SNPs, 28,850 genes for RNA-seq data and 748 metabolite for metabolome data for each individual. The data associated with these features were scaled to mean 0 with unit variance before the multi-omics were integrated into the autoencoder algorithm. Three parameters were set as following: 1024 nodes for 1 hidden layer and 70 latent factors with 2,000 epochs for the unsupervised deep learning training for the autoencoder. The reconstructed multi-omics from this unsupervised learning process is applied to ridge regression prediction model for prediction performance evaluation in comparison with SNP data only.

Best linear unbiased predictions (BLUP) of three breeding traits (i.e. ear diameter, silking time, pollen shedding time) were included to compare the multi-omics-based trait prediction with the SNP-based method based on testing set (10% of whole panel) under the same ridge regression supervised learning algorithm trained with the training set (90% of whole panel). Each comparison for each trait was repeated for 100 times of randomized training-testing split.

With SNP derived ridge regression prediction, the mean of prediction accuracy based on the testing set was 0.54, 0.63, 0.47 for silking time, pollen shedding time and ear diameter, respectively. By integrating additional layer of omics layers (i.e. transcriptome and metabolome data) in this example, the mean of prediction accuracy improves for silk time by 19.4% from 0.54 to 0.64 (p-value from paired t-test: 1.6E-08), for pollen shedding time by 10% from 0.63 to 0.69 (p-value: 2.1E-05) and for ear diameter by 13.4% from 0.47 to 0.54 (p-value: 0.004) as compared to the prediction accuracy by SNP only.

Thus, this example demonstrates the benefit of multi-omics integration for breeding trait prediction compared to a genetic variation (e.g. SNPs) derived prediction. In addition, the multi-omics data types are not limited to the ones in the current examples. For example, in an embodiment, the multi-omics data type can be hyperspectral images collected from precision agriculture fields.

### EXAMPLE 2: Integrative Multi-omics Application with Hyperspectral Imaging in Plant Breeding

The development of hyperspectral imaging leads to many advances in precision agriculture through different applications, such as monitoring plant drought stress, plant disease and nutrient stress etc. Hyperspectral imaging captures and processes an image at a large number of wavelengths. Thus, a hyperspectral image includes tens and hundreds of data points across all the wavelengths as features for a phenome.

This example stated here is to demonstrate how the phenome data captured by the hyperspectral imaging can be integrated with other omics data for plant breeding applications. In Example 1, it is illustrated that the hyperspectral image data is collected for each of the 339 maize lines in addition to the SNP, RNA-seq and metabolome data described already. Among the phenome data, there are 800 spectral wavelengths beside 72,572 SNPs, 28,850 genes for RNA-seq data and 748 metabolites for metabolome data for each individual. These 800 features derived from the hyperspectral imaging can be scaled to mean 0 with unit variance with other multi-omics before being integrated into the autoencoder algorithm. Three parameters can be set as following: 1024 nodes for 1 hidden layer and 70 latent factors with 2,000 epochs for the unsupervised deep learning training for the autoencoder. The reconstructed multi-omics from this unsupervised learning process is applied to ridge regression prediction model for prediction performance evaluation in comparison with SNP data only.

The multi-omics integration can be done across SNP, transcriptome, metabolome and phenome together described above or can include the phenome with additional single layer of omics type, such as SNP, transcriptome, or metabolome alone. Furthermore, the phenome data on the same population can be collected across different environments for multi-omics data integration to predict the phenotypes under different environments. In addition, this multi-omics data integration framework with hyperspectral imaging data can be further expanded across multiple breeding population for phenotype prediction.

### EXAMPLE 3: Integrative Multi-omics Application with NIR, Hyperspectral Imaging, and SNPs in Plant Breeding

The example stated here is to demonstrate how hyperspectral imaging data can be integrated with other omics data for plant breeding applications using a hybrid machine learning framework. The hybrid framework of unsupervised and supervised learning illustrated in FIG. 4 was applied to a total of 761 Corteva maize lines with SNP and hyperspectral imaging data. In this example, hyperspectral imaging data was collected for each of the 761 maize lines at individual seed level with NIR technology with 785 wavelengths and seedling stages at two time points using THEIA technology with 400 wavelengths. For SNP data, 1003 SNPs were used for genotyping each maize line. These features were scaled to mean 0 with unit variance before the multi-omics were integrated into the autoencoder algorithm. Three parameters were set as following: 1024 nodes for 1 hidden layer and 70 latent factors with 2,000 epochs for the unsupervised deep learning training for the autoencoder. The reconstructed multi-omics from this unsupervised learning process was applied to ridge regression prediction model for prediction performance evaluation in comparison with SNP data only.

Best linear unbiased predictions (BLUP) of 16 breeding traits (e.g., yield, moisture, plant height) were included to compare the multi-omics-based trait prediction with the SNP-based method based on testing set (10% of whole panel) under the same ridge regression supervised learning algorithm trained with the training set (90% of whole panel). Each comparison for each trait was repeated for 100 times of randomized training-testing split.
The comparison between SNP and multi-omics-based selection accuracy is summarized in Table 1. Among the 16 different traits, the proposed hybrid framework can improve the selection accuracy by 45% (pvalue: 4.75e-31) compared to SNP only based prediction method. Accuracy was calculated using the correlation between the predicted value and observed value for a specific trait.

Thus, use of an unsupervised and supervised learning framework with multi-omics data integration, such as from SNPs, NIR, and hyperspectral imaging data, can be used to predict phenotypes and aid in the selection or culling of certain traits or plants in a breeding program. In some instances, this approach increases the accuracy of the phenotypes predicted compared to a single omics approach, such as SNPs alone.

**Table 1: Comparison of selection accuracy between SNP and multi-omics methods**

| Phenotype | SNP-Cross Validation (CV) | SNP+ NIR+Seedling Hyperspectral Imaging (HI)-Cross Validation (CV) | Accuracy Increase (%) | p-value |
|---|---|---|---|---|
| | Accuracy | | | |
| | | Accuracy | | |
| Yield (bu/a) | 0.45 | 0.56 | 25 | 3.64E-20 |
| Plant Height (inches) | 0.50 | 0.60 | 20 | 6.71E-16 |
| Test Weight (lb/bu) | 0.61 | 0.64 | 6 | 1.14E-04 |
| Moisture (%) | 0.50 | 0.63 | 27 | 5.28E-30 |
| Ear Height (inches) | 0.49 | 0.57 | 15 | 4.02E-11 |
| Predicted Relative Maturity (Day) | 0.49 | 0.62 | 26 | 2.23E-27 |
| Yield Moisture (value) | 0.40 | 0.46 | 13 | 5.31E-06 |
| Appearance of hybrids (score) | 0.57 | 0.65 | 15 | 4.67E-13 |
| Plant Height Covariate Yield (bu/a) | 0.47 | 0.58 | 22 | 1.72E-21 |
| Remote Sensing Stay Green (%) | 0.59 | 0.73 | 24 | 1.62E-34 |
| Remote Sensing Canopy Cover (%) | 0.40 | 0.58 | 45 | 4.75E-31 |
| Remote Sensing Hue of Green Color (%) | 0.50 | 0.66 | 31 | 4.13E-36 |
| Remote Sensing Canopy Health(%) | 0.44 | 0.64 | 44 | 1.62E-38 |
| Remote Sensing Plot Score Green Plant Cover (%) | 0.40 | 0.52 | 29 | 1.84E-17 |
| Remote Sensing Plot Score Green Hue (%) | 0.54 | 0.70 | 29 | 1.06E-33 |
| Adjusted growth income ($ one would make on the crop minus the cost of drying it down) | 0.42 | 0.51 | 22 | 1.03E-13 |

## Claims

1. A computer-implemented method of predicting at least one phenotype of interest for one or more plants, the method comprising:
(a) generating a universal integrated latent space representation by encoding variables derived from two or more types of data obtained from training and testing plant populations into latent vectors through a machine learning-based multi-modal variational autoencoder framework, wherein the two or more types of data comprise genomic data, exomic data, epigenomic data, transcriptomic data, proteomic data, metabolomic data, hyperspectral data, or phenomic data, or combinations thereof and wherein the latent space is independent of the underlying genomic, exomic, epigenomic, transcriptomic, proteomic, metabolomic, hyperspectral, or phenomic association;
(b) decoding the integrated latent representation by a decoder to obtain reconstructed data for the training and testing plant populations;
(c) inputting the reconstructed data from the training plant population and observed phenotype data for at least one phenotype of interest obtained from the training plant population to train a supervised learning model;
(d) predicting the at least one phenotype of interest for one or more plants from the testing population by inputting the reconstructed data for the testing population into the trained supervised learning model; and
(e) selecting the one or more plants from the testing population based on at least one predicted phenotype of interest.

2. The method of claim 1:
(I) further comprising growing the selected one or more plants in a plant growing environment;
(II) wherein the variables are discrete variables, continuous variables, or both;
(III) wherein the generated a universal integrated latent space representation is a universal continuous integrated latent space representation;
(IV) wherein the at least one predicted phenotype and observed phenotype of interest is an agronomic phenotype;
(V) wherein the at least one predicted phenotype and observed phenotype of interest is a breeding trait;
(VI) wherein the at least one predicted phenotype and observed phenotype of interest is yield, root lodging, stalk lodging, brittle snap, ear height, grain moisture, plant height, disease resistance, or abiotic stress tolerance;
(VII) wherein the selected plants exhibit an improved or increased at least one predicted phenotype of interest compared to a control; or
(VIII) wherein the accuracy of phenotype prediction for the at least one predicted phenotype is improved compared to the accuracy of phenotype prediction for the at least one predicted phenotype for a control.

3. The method of claim 1, wherein the two or more types of data comprises genomic-wide data, exomic-wide data, epigenomic-wide data, transcriptomic-wide data, proteomic-wide data, metabolomic-wide data, or phenomic-wide data; preferably wherein:
(i) the genomic-wide data comprises a collection of alleles, haplotypes, genotypic markers, indels, single nucleotide polymorphisms, or combinations thereof;
(ii) the exomic-wide data comprises a collection of exome DNA sequences;
(iii) the epigenomic-wide data comprises gene expression, chromatin accessibility, DNA methylation, histone modifications, recombination hotspot, genomic landing locations for transgenes, transcription factor binding status, or any combination thereof;
(iv) the transcriptomic-wide data comprises a collection of RNA transcript sequences and profile information;
(v) the proteomic-wide data comprises a collection of protein sequences and profile information; or
(vi) the metabolomic-wide data comprises a collection of metabolites and profile information.

4. The method of claim 1, wherein the hyperspectral data comprises RGB imaging data, infrared imaging data, or spectral imaging data, or any combinations thereof; preferably wherein:
(i) the infrared imaging data comprises NIR, FIR, or thermal infrared imaging data or any combinations thereof; or
(ii) the spectral imaging comprises hyperspectral or multispectral imaging data or any combinations thereof.

5. A computing device comprising a processor configured to perform the steps of a-d in the method of claim 1.

6. A computer-readable medium comprising instructions which, when executed by a computing device, cause the computing device to carry out the steps of a-d in the method of claim 1.

## Patentansprüche

1. Computerimplementiertes Verfahren zum Vorhersagen mindestens eines Phänotyps von Interesse für eine oder mehrere Pflanzen, wobei das Verfahren Folgendes umfasst:
(a) Erzeugen einer universellen, integrierten Latentraumdarstellung durch Codieren von Variablen, die aus zwei oder mehr Arten von Daten abgeleitet sind, welche aus Trainings- und Testpflanzenpopulationen erhalten wurden, in Latentvektoren mittels eines auf maschinellem Lernen basierenden multimodalen variationalen Autoencoder-Frameworks, wobei die zwei oder mehr Arten von Daten Genomdaten, Exomdaten, Epigenomdaten, Transkriptomdaten, Proteomdaten, Metabolomdaten, Hyperspektraldaten oder Phänotypdaten oder Kombinationen davon umfassen und wobei der Latenzraum unabhängig von der zugrunde liegenden genomischen, exomischen, epigenomischen, transkriptomischen, proteomischen, metabolomischen, hyperspektralen oder phänotypischen Assoziation ist;
(b) Decodieren der integrierten latenten Darstellung durch einen Decoder, um rekonstruierte Daten für die Trainings- und Testpflanzenpopulationen zu erhalten;
(c) Eingeben der rekonstruierten Daten aus der Trainingspflanzenpopulation und der beobachteten Phänotypdaten für mindestens einen Phänotyp von Interesse, der aus der Trainingspflanzenpopulation erhalten wurde, um ein überwachtes Lernmodell zu trainieren;
(d) Vorhersagen des mindestens einen Phänotyps von Interesse für eine oder mehrere Pflanzen aus der Testpopulation durch Eingeben der rekonstruierten Daten für die Testpopulation in das trainierte überwachte Lernmodell; und
(e) Auswählen der einen oder mehreren Pflanzen aus der Testpopulation basierend auf mindestens einem vorhergesagten Phänotyp von Interesse.

2. Verfahren nach Anspruch 1:
(I) das ferner das Züchten der ausgewählten einen oder mehreren Pflanzen in einer Pflanzenwachstumsumgebung umfasst;
(II) wobei die Variablen diskrete Variablen, kontinuierliche Variablen oder beides sind;
(III) wobei die erzeugte eine universelle integrierte Latentraumdarstellung eine universelle kontinuierliche integrierte Latentraumdarstellung ist;
(IV) wobei der mindestens eine vorhergesagte Phänotyp und der beobachtete Phänotyp von Interesse ein agronomischer Phänotyp ist;
(V) wobei der mindestens eine vorhergesagte Phänotyp und der beobachtete Phänotyp von Interesse ein Zuchtmerkmal ist;
(VI) wobei der mindestens eine vorhergesagte und beobachtete Phänotyp von Interesse der Ertrag, die Wurzel- oder Stängelknickung, das Sprödebrechen, die Ährenhöhe, die Kornfeuchte, die Pflanzenhöhe, die Krankheitsresistenz oder die Toleranz gegenüber abiotischem Stress ist;
(VII) wobei die ausgewählten Pflanzen einen verbesserten oder erhöhten mindestens einen vorhergesagten Phänotyp von Interesse im Vergleich zu einer Kontrolle zeigen; oder
(VIII) wobei die Genauigkeit der Phänotypvorhersage für den mindestens einen vorhergesagten Phänotyp im Vergleich zu der Genauigkeit der Phänotypvorhersage für den mindestens einen vorhergesagten Phänotyp für eine Kontrolle verbessert ist.

3. Verfahren nach Anspruch 1, wobei die zwei oder mehr Datentypen genomweite Daten, exomweite Daten, epigenomweite Daten, transkriptomweite Daten, proteomweite Daten, metabolomweite Daten oder phänotypomweite Daten umfassen; vorzugsweise wobei:
(i) die genomweiten Daten eine Sammlung von Allelen, Haplotypen, genotypischen Markern, Indels, Einzelnukleotid-Polymorphismen oder Kombinationen davon umfassen;
(ii) die exomweiten Daten eine Sammlung von Exom-DNA-Sequenzen umfassen;
(iii) die epigenomweiten Daten Genexpression, Chromatinzugänglichkeit, DNA-Methylierung, Histonmodifikationen, Rekombinations-Hotspots, genomische Einlagerungsorte für Transgene, den Bindungsstatus von Transkriptionsfaktoren oder eine beliebige Kombination davon umfassen;
(iv) die transkriptomweiten Daten eine Sammlung von RNA-Transkriptsequenzen und Profilinformationen umfassen;
(v) die proteomweiten Daten eine Sammlung von Proteinsequenzen und Profilinformationen umfassen; oder
(vi) die metabolomweiten Daten eine Sammlung von Metaboliten und Profilinformationen umfassen.

4. Verfahren nach Anspruch 1, wobei die Hyperspektraldaten RGB-Bildgebungsdaten, Infrarotbildgebungsdaten oder Spektralbildgebungsdaten oder beliebige Kombinationen davon umfassen; vorzugsweise wobei:
(i) die Infrarotbilddaten NIR-, FIR- oder thermische Infrarotbilder oder beliebige Kombinationen davon umfassen; oder
(ii) die spektrale Bildgebung hyperspektrale oder multispektrale Bilddaten oder beliebige Kombinationen davon umfasst.

5. Rechenvorrichtung, die einen Prozessor umfasst, der so konfiguriert ist, dass er die Schritte a bis d des Verfahrens nach Anspruch 1 ausführt.

6. Computerlesbares Medium, das Anweisungen enthält, die, wenn sie von einer Rechenvorrichtung ausgeführt werden, bewirken, dass die Rechenvorrichtung die Schritte a bis d des Verfahrens nach Anspruch 1 ausführt.

## Revendications

1. Procédé mis en œuvre par ordinateur de prédiction d'au moins un phénotype d'intérêt pour une ou plusieurs plantes, le procédé comprenant :
(a) la génération d'une représentation universelle intégrée de l'espace latent en codant des variables dérivées d'au moins deux types de données obtenues à partir de l'entraînement et de l'essai de populations de plantes en vecteurs latents par l'intermédiaire d'un cadre d'autocodeur variationnel multimodal basé sur l'apprentissage automatique, dans lequel les au moins deux types de données comprennent des données génomiques, des données exomiques, des données épigénomiques, des données transcriptomiques, des données protéomiques, des données métabolomiques, des données hyperspectrales ou des données phénomiques, ou leurs combinaisons, et dans lequel l'espace latent est indépendant de l'association génomique, exomique, épigénomique, transcriptomique, protéomique, métabolomique, hyperspectrale ou phénomique sous-jacente ;
(b) le décodage de la représentation latente intégrée par un décodeur afin d'obtenir des données reconstruites pour les populations de plantes d'entraînement et d'essai ;
(c) l'entrée des données reconstruites à partir de la population de plantes d'entraînement et des données de phénotype observé pour au moins un phénotype d'intérêt obtenu à partir de la population de plantes d'entraînement afin d'entraîner un modèle d'apprentissage supervisé ;
(d) la prédiction du ou des phénotypes d'intérêt pour une ou plusieurs plantes provenant de la population testée en entrant les données reconstruites pour la population testée dans le modèle d'apprentissage supervisé entraîné ; et
(e) la sélection de la ou des plantes parmi la population testée sur la base d'au moins un phénotype d'intérêt prédit.

2. Procédé selon la revendication 1 :
(I) comprenant en outre la culture de la ou des plantes sélectionnées dans un environnement de culture de plantes ;
(II) dans lequel les variables sont des variables discrètes, des variables continues ou les deux ;
(III) dans lequel la représentation universelle intégrée d'espace latent générée est une représentation universelle intégrée continue d'espace latent ;
(IV) dans lequel le ou les phénotypes prédits et le phénotype d'intérêt observé sont des phénotypes agronomiques ;
(IV) dans lequel le ou les phénotypes prédits et le phénotype d'intérêt observé sont des caractères de sélection ;
(VI) dans lequel le ou les phénotypes prédits et le phénotype d'intérêt observé sont le rendement, la verse des racines, la verse des tiges, le détachement en vert, la hauteur des épis, l'humidité des grains, la hauteur des plantes, la résistance aux maladies ou la tolérance au stress abiotique ;
(VII) dans lequel les plantes sélectionnées présentent au moins un phénotype d'intérêt prédit amélioré ou augmenté par rapport à un témoin ; ou
(VIII) dans lequel la précision de la prédiction de phénotype pour le ou les phénotypes prédits est améliorée par rapport à la précision de la prédiction de phénotype pour le ou les phénotypes prédits pour un témoin.

3. Procédé selon la revendication 1, dans lequel les au moins deux types de données comprennent des données à l'échelle génomique, des données à l'échelle exomique, des données à l'échelle épigénomique, des données à l'échelle transcriptomique, des données à l'échelle protéomique, des données à l'échelle métabolomique ou des données à l'échelle phénomique ; de préférence dans lequel :
(i) les données à l'échelle génomique comprennent une collection d'allèles, d'haplotypes, de marqueurs génotypiques, d'indels, de polymorphismes à nucléotide unique, ou leurs combinaisons ;
(ii) les données à l'échelle exomique comprennent une collection de séquences d'ADN d'exome ;
(iii) les données à l'échelle épigénomique comprennent l'expression génique, l'accessibilité à la chromatine, la méthylation de l'ADN, les modifications des histones, un point chaud de recombinaison, des emplacements d'atterrissage génomique pour les transgènes, l'état de liaison aux facteurs de transcription ou toute combinaison de ceux-ci ;
(iv) les données à l'échelle transcriptomique comprennent une collection de séquences de transcription d'ARN et d'informations de profil ;
(v) les données à l'échelle protéomique comprennent une collection de séquences protéiques et d'informations de profil ; ou
(vi) les données à l'échelle métabolomique comprennent une collection de métabolites et d'informations de profil.

4. Procédé selon la revendication 1, dans lequel les données hyperspectrales comprennent des données d'imagerie RGB, des données d'imagerie infrarouge, ou des données d'imagerie spectrale, ou toute combinaison de celles-ci ; de préférence dans lequel :
(i) les données d'imagerie infrarouge comprennent des données NIR, FIR ou des données d'imagerie infrarouge thermique ou toute combinaison de celles-ci ; ou
(ii) l'imagerie spectrale comprend des données d'imagerie hyperspectrale ou multispectrale ou toute combinaison de celles-ci.

5. Dispositif informatique comprenant un processeur configuré pour exécuter les étapes a à d du procédé selon la revendication 1.

6. Support lisible par ordinateur comprenant des instructions qui, lorsqu'elles sont exécutées par un dispositif informatique, amènent le dispositif informatique à exécuter les étapes a à d du procédé selon la revendication 1.
